# EUROPÄISCHE PATENTSCHRIFT

(11) **EP 0 958 781 B1**
(45) Veröffentlichungstag und Bekanntmachung des Hinweises auf die Patenterteilung: **03.03.2004**
(21) Anmeldenummer: 99109987.0
(22) Anmeldetag: 21.05.1999
(51) Int. Cl.: A61B 5/042

(54) **Vorrichtung zur Lagekontrolle eines Venen-Katheters mittels EKG-Ableitung**
Device for controlling position of a venous catheter by ECG
Dispositif pour contrôler la position d'un cathéter veineux par ECG

(30) Priorität: 23.05.1998 DE 19823146
(43) Veröffentlichungstag der Anmeldung: 24.11.1999
(73) Patentinhaber: Pajunk, Heinrich, 78187 Geisingen (DE); Pajunk, Horst, 78187 Geisingen (DE)
(72) Erfinder: Pajunk, Heinrich, 78187 Geisingen (DE); Pajunk, Horst, 78187 Geisingen (DE)
(74) Vertreter: Patentanwälte Westphal, Mussgnug & Partner

(56) Entgegenhaltungen:
- DE-A- 4 422 455
- DE-C- 4 318 963
- US-A- 4 644 960

## Beschreibung

Die Erfindung betrifft eine Vorrichtung zur Lagekontrolle eines Venen-Katheters mittels EKG-Ableitung gemäß dem Oberbegriff des Anspruchs 1.

Beim Einsatz von Venen-Kathetern (Cava-Kathetern) ist eine gute Lagekontrolle der distalen Spitze des Katheters für die Sicherheit der Methode unerläßlich.

Die herkömmliche Lagekontrolle mittels Röntgenstrahlen stellt eine erhebliche Belastung für den Patienten dar, einerseits durch die Röntgenstrahlen und andererseits durch die Injektion des Kontrastmittels. Darüberhinaus ist die Röntgenmethode kostenaufwendig.

Diese Belastungen vermeidet die gattungsgemäße Vorrichtung, bei welcher die Lage der distalen Spitze des Katheters dadurch kontrolliert wird, daß eine elektrisch leitende Flüssigkeit in dem Katheter zur EKG-Ableitung verwendet wird. Die Flüssigkeitssäule in dem Katheter bildet einen elektrischen Leiter für die EGK-Ableitung, der nur an dem distalen Austrittsende des Katheters nicht isoliert ist. Als elektrisch leitende Flüssigkeit kann eine Elektrolytlösung, z. B. eine physiologische Kochsalzlösung, oder das Blut selbst verwendet werden. Bei der intraatrialen EKG-Ableitung wird dabei zur Lokalisierung der Katheterspitze die Erscheinung ausgenutzt, daß die P-Welle des EKG bei Übergang von der Vena Cava in den Vorhof des Herzens eine charakteristische Überhöhung zeigt. Der Katheter wird soweit in die Vena Cava eingeführt, bis sich die Katheterspitze im rechten Vorhof des Herzens befindet, was durch die überhöhte P-Welle erkennbar ist. Der Katheter wird dann langsam aus dem rechten Vorhof zurückgezogen, bis sich die P-Welle wieder normalisiert. Nach einem weiteren Zurückziehen um 1 bis 3 cm hat der Katheter seine optimale Position erreicht und kann in dieser Lage fixiert werden.

Um die Patientenleitung des EKG-Gerätes elektrisch leitend mit der Flüssigkeitssäule in dem Venen-Katheter zu verbinden, ist es zum Beispiel aus der DE 82 29 899 U1 bekannt, zwischen die Spritze zum Einleiten der leitenden Flüssigkeit und das proximale Ende des Katheters eine Verbindungshülse einzusetzen, in deren Durchgangskanal ein Kontakt eingesetzt ist, der mit einem Anschluss für die Patientenleitung verbunden ist. Die Konnektierungen der Verbindungshülse mit dem Katheter einerseits und mit der Spritze andererseits können sich unbeabsichtigt lösen. Nach der Positionierung der Katheterspitze muß die Verbindungshülse entfernt werden. Das Entfernen der Verbindungshülse und das Anschließen der Infusionsleitung können ein Risiko für Luftembolien bilden.

Um diese Risiken zu verringern, ist bei einer bekannten Vorrichtung der eingangs genannten Gattung am distalen Ende des Katheters ein Drei-Wegehahn anschließbar, über welchen umschaltbar sowohl eine Infusionsleitung als auch eine Spritze für die leitfähige Flüssigkeit an den Katheter angeschlossen werden kann. Der Kontakt für den Anschluß der Patientenleitung des EKG-Gerätes kann auch hierbei in einer zusätzlichen Verbindungshülse angeordnet sein oder ist gemäß der EP 0 153 952 B1 in dem Anschlußkonus der Spritze eingesetzt. Beim Umschalten von der Lagekontrolle mittels EKG-Ableitung zur Infusionsleitung ist es nicht erforderlich, den Katheter zu dekonnektieren. Die Verbindungshülse muß entfernt werden oder es ist erforderlich eine spezielle Spritze zu verwenden, die den Anschluß für das EKG-Gerät enthält. Eine solche Spritze ist in der Herstellung aufwendig. Das Sterilisieren ist wegen des in die Spritze eingesetzten Kontaktes schwierig.

Der Erfindung liegt die Aufgabe zugrunde, eine Vorrichtung zur Lagekontrolle eines Venen-Katheters mittels EKG-Ableitung zur Verfügung zu stellen, die einfach aufgebaut, betriebssicher und kostengünstig herstellbar ist.

Diese Aufgabe wird erfindungsgemäß gelöst durch eine Vorrichtung mit den Merkmalen des Anspruchs 1.

Vorteilhafte Ausführungen der Erfindung sind in Unteransprüchen angegeben.

Der Erfindung liegt der Gedanke zugrunde, den Kontakt für die leitende Flüssigkeit in das Hahnküken des Drei-Wegehahnes einzusetzen. Dadurch sind keine zusätzlichen Adapterteile für den EKG-Anschluß erforderlich. Beim Umschalten von der Lagekontrolle auf die Infusion muß kein Anschluß dekonnektiert werden, so daß das Risiko von Luftembolien ausgeschlossen ist. Der Kontakt für den EKG-Anschluß ist in den Drei-Wegehahn integriert, so daß sich keine zusätzliche Möglichkeit für den Eintritt von Infektionskeimen oder dergleichen ergibt. Die Vorrichtung bedeutet nur eine geringfügige Modifikation des ohnehin benötigten Drei-Wegehahnes, so daß sich eine preisgünstige Herstellung und damit eine erhebliche Kostenreduzierung ergibt.

Vorzugsweise ist unmittelbar an dem Drei-Wegehahn ein Steckverbinder für das Anschließen der Patientenleitung angebracht. Die Patientenleitung kann nach der Positionierung des Katheters einfach abgezogen werden und kann jederzeit wieder aufgesteckt werden, falls eine erneute Lagekontrolle notwendig werden sollte. Nach dem Entfernen der Patientenleitung vergrößert der Steckverbinder die Abmessungen des Drei-Wegehahnes praktisch nicht, so daß ein einfaches Fixieren des Drei-Wegehahnes am Körper des Patienten auch über eine längere Zeitdauer möglich ist.

In einer zweckmäßigen Ausführung weist das Hahnküken des Drei-Wegehahnes eine achsparallele Bohrung auf, in welche ein Kontaktteil eingesetzt ist, das an seinem einen Ende den Steckerstift des Steckverbinders für den Anschluß der Patientenleitung bildet und welches mit seinem anderen Ende in den Durchgang des Hahnkükens hineinragt. Das Kontaktteil weist Dichtungen auf, um den Durchgang des Hahnkükens nach außen abzudichten.

Im folgenden wird die Erfindung anhand in der Zeichnung dargestellter Ausführungsbeispiele näher erläutert. Es zeigen:
- Figur 1: eine Seitenansicht des Drei-Wegehahnes in einer ersten Ausführung, wobei die untere Hälfte axial in der Achsebene des Hahnkükens geschnitten ist, und
- Figur 2: eine Seitenansicht des Drei-Wegehahnes in Achsrichtung des Hahnkükens gesehen, wobei ebenfalls die untere Hälfte axial geschnitten ist,
- Figur 3: das Hahnküken des Drei-Wegehahnes in einer in der unteren Hälfte axial geschnittenen Seitenansicht,
- Figur 4: das Kontaktteil dieser ersten Ausführung,
- Figur 5: eine Figur 1 entsprechende Darstellung einer zweiten Ausführung des Drei-Wegehahnes und
- Figur 6: eine in der unteren Hälfte axial geschnittene Seitenansicht des Kontaktteiles dieser zweiten Ausführung.

Die Vorrichtung weist einen Drei-Wegehahn auf, dessen Körper 10 aus Kunststoff besteht. An dem Körper 10 des Drei-Wegehahnes sind drei Anschlüsse angeformt, nämlich ein Anschluß 12 für einen Venen-Katheter, der als männlicher Luer-Lock-Ansatz ausgebildet ist, ein Anschluß 14 mit einem weiblichen Konus zum Anschließen einer Infusionsleitung und ein Anschluß 16 zum Anschließen einer Spritze zum Aspirieren von Blut und zum Einleiten einer elektrisch leitenden Flüssigkeit.

In den kreiszylindrischen Innenraum des Körpers 10 ist ein zylindrisches Hahnküken 18 eingesetzt, welches an seinem Mantelumfang drei Durchgangsöffnungen 20 aufweist, deren Anordnung der Anordnung der Anschlüsse 12, 14 und 16 entspricht. Das Hahnküken 18 besteht ebenfalls aus Kunststoff und ist mittels eines Ringwulstes 21 abgedichtet in den Körper 10 eingeschnappt, so daß das Hahnküken 18 in dem Körper 10 axial festgelegt und um seine Achse drehbar ist. Zum Verdrehen des Hahnkükens 18 ist dieses mit Griffflügeln 22 versehen, die gleichzeitig die Winkelposition der Durchgangsöffnungen 20 des Hahnkükens 18 für den Benutzer anzeigen.

Das Hahnküken 18 weist eine koaxial durchgehende Innenbohrung 24 auf, in welche die Durchgangsöffnungen 20 münden. In die Innenbohrung 24 ist ein metallisches, elektrisch leitendes Kontaktteil 26 koaxial eingesetzt.

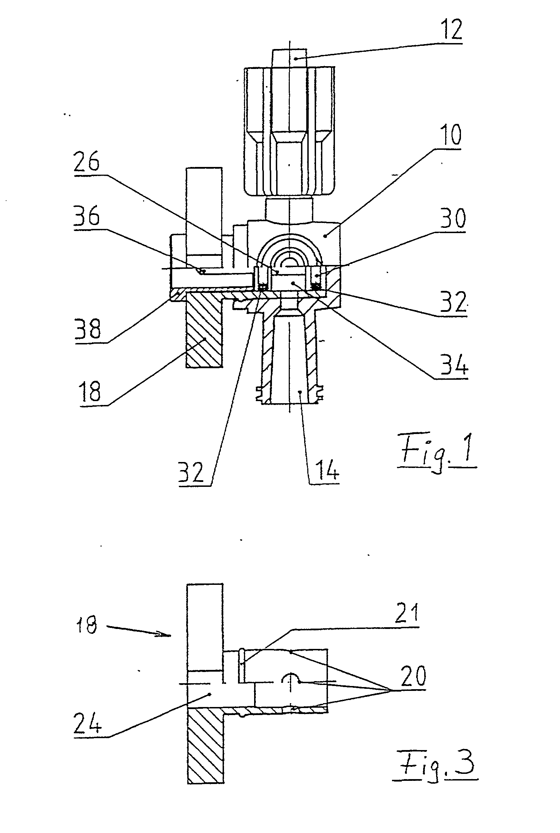

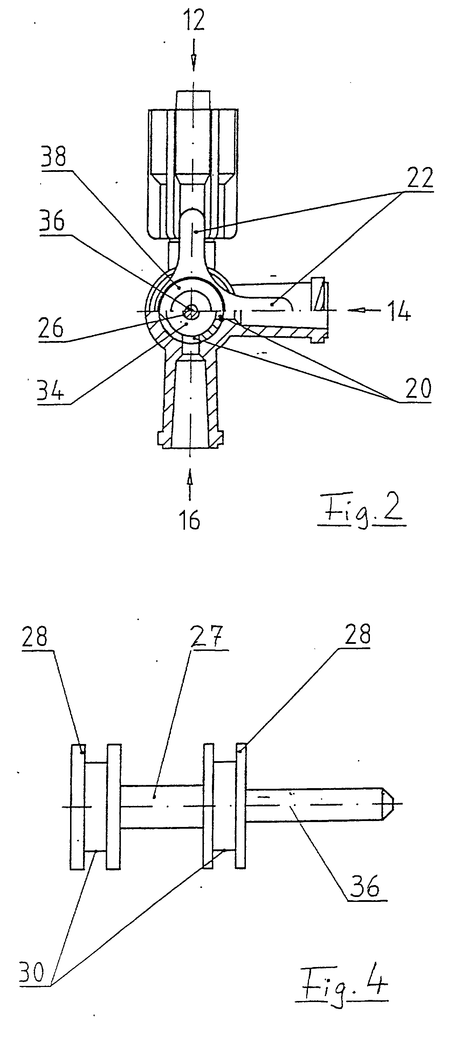

Das Kontaktteil 26 besteht in dem ersten Ausführungsbeispiel der Figuren 1 bis 4 aus einem achsmittig in der Innenbohrung 24 angeordneten Stift 27, der einstückig mit zwei axial beabstandeten Bundscheiben 28 größeren Durchmessers ausgebildet ist. Die Bundscheiben 28 halten den Stift 27 zentriert in der Innenbohrung 24 und stützen diesen radial in der Innenbohrung 24 ab. In einer Umfangsnut 30 der Bundscheiben 28 ist jeweils ein O-Ring 32 eingesetzt, der die Bundscheiben 28 gegen die Innenwandung der Innenbohrung 24 abdichtet. Die Bundscheiben 28 sind axial so an dem Kontaktteil 26 angeordnet, daß jeweils eine der Bundscheiben 28 oberhalb und die andere unterhalb der Durchgangsöffnungen 20 sitzt. Der Stift 27 und die Bundscheiben 28 bilden somit eine Ringkammer 34, die mit den Durchgangsöffnungen 20 in Verbindung steht und durch die O-Ringe 32 axial abgedichtet ist. Die eine Bundscheibe 28 sitzt auf dem inneren Boden des Körpers 10 auf. Über die andere Bundscheibe 28 hinaus setzt sich das Kontatktteil 26 einstückig in einem axialen Steckerstift 36 eines Steckverbinders zum Anschließen der Patientenleitung eines EKG-Gerätes fort. Das Kontaktteil 26 ist vergoldet, um einerseits einen guten Kontakt des Stekkerstifts 36 zu gewährleisten und um andererseits eine Korrosion des Kontaktteiles 26 durch die Elektrolytflüssigkeit zu verhindern.

Nach dem Einsetzen des Kontaktteiles 26 in die Innenbohrung 24 des Hahnkükens 18 wird eine Führungsbuchse 38 aus Kunststoff in die Innenbohrung 24 eingepresst, die einerseits das Kontaktteil 26 axial in dem Hahnküken 18 festlegt und die andererseits eine Führung für die Steckverbinder-Buchse der Patientenleitung beim Aufstecken auf den Steckerstift 36 bildet.

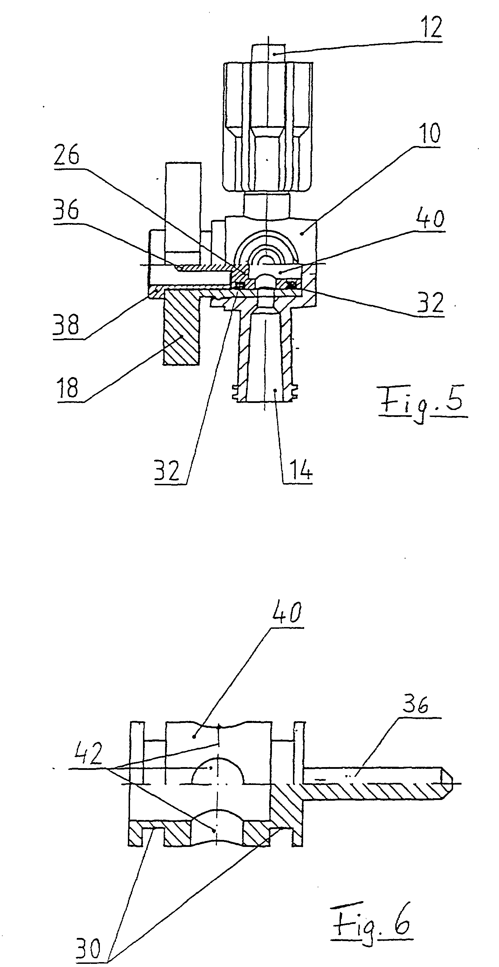

Die Figuren 5 und 6 zeigen eine zweite Ausführung des Drei-Wegehahnes. Soweit diese Ausführung mit der ersten Ausführung übereinstimmt, werden die gleichen Bezugszeichen verwendet und auf die vorstehende Beschreibung wird Bezug genommen.

Der Körper 10 des Drei-Wegehahnes mit den Anschlüssen 12, 14 und 16 sowie das Hahnküken 18 stimmen mit dem ersten Ausführungsbeispiel der Figuren 1 bis 4 überein. Das Kontaktteil 26 ist dagegen, wie aus Figur 6 ersichtlich ist, mit einem Hohlzylinder 40 ausgebildet, der in die Innenbohrung 24 des Hahnkükens 18 eingesetzt ist. Der Außendurchmesser des Hohlzylinders 40 entspricht dem Innendurchmesser der Innenbohrung 24. Der Mantel des Hohlzylinders 40 ist von Durchgangsöffnungen 42 durchbrochen, die in ihrer axialen Anordnung, ihrer Winkelanordnung und in ihrem Durchmesser den Durchgangsöffnungen 20 des Hahnkükens 18 entsprechen. An dem auf dem Boden der Innenbohrung 24 des Hahnkükens 18 aufsitzendem Ende ist der Hohlzylinder 40 offen. Das entgegengesetzte Ende des Hohlzylinders 40 ist geschlossen. An diesem geschlossenen Ende setzt einstückig axial mittig der Steckerstift 36 an. An den beiden axialen Enden des Hohlzylinders 40 sind in dessen äußerer Mantelfläche Umfangsnuten 30 eingestochen, in welche O-Ringe 32 zur Abdichtung eingelegt werden.

Wird das in Figur 6 dargestellte Kontaktteil 26 in die Innenbohrung 24 des Hahnkükens 18 eingesetzt, wie dies in Figur 5 gezeigt ist, so sitzt der Hohlzylinder 40 mit seinem offenen Ende auf dem Boden der Innenbohrung 24 des Hahnkükens 18 auf. Die Durchgangsöffnungen 42 des Hohlzylinders 40 kommen mit den Durchgangsöffnungen 20 des Hahnkükens 18 zur Deckung, so daß die elektrisch leitende Flüssigkeit über den Anschluß 14 durch die Durchgangsöffnungen 20 und 42 in das Innere des Hohlzylinders 40 und von dort in den Anschluß 12 für den Venen-Katheter gelangt. Die Flüssigkeit steht mit dem elektrisch leitenden Hohlzylinder 40 in Berührung und damit in elektrisch leitendem Kontakt mit dem Steckerstift 36. Die O-Ringe 32 dichten das Kontaktteil 26 ab, so daß die Flüssigkeit nicht aus dem Hahnküken 18 austreten kann.

Beim Positionieren der distalen Katheterspitze wird zur Lagekontrolle eine elektrisch leitende Flüssigkeit, zum Beispiel eine physiologische Kochsalzlösung mittels einer Spritze über den Anschluß 16 in den an den Anschluß 12 konnektierten Venen-Katheter eingeleitet. Die elektrisch leitende Flüssigkeit fließt dabei durch das Hahnküken 18, steht in Kontakt mit dem Kontaktteil 26 und kann über den Steckerstift 36 in leitende Verbindung mit der Patientenleitung des EKG-Gerätes gebracht werden. Ist die Katheterspitze positioniert, so kann der Drei-Wegehahn umgeschaltet werden, so daß die an den Anschluß 14 konnektierte Infusionsleitung mit dem Venen-Katheter in Verbindung steht. Die Patientenleitung des EKG-Gerätes kann nun von dem Steckerstift 36 abgezogen werden.

### Bezugszeichenliste

- 10: Körper
- 12: Anschluß für Venen-Katheter
- 14: Anschluß für Infusion
- 16: Anschluß für Spritze
- 18: Hahnküken

- 20: Durchgangsöfnungen
- 21: Ringwulst
- 22: Grifflügel
- 24: Innenbohrung
- 26: Kontaktteil
- 27: Stift
- 28: Bundscheiben

- 30: Umfangsnut
- 32: O-Ring
- 34: Ringkammer
- 36: Steckerstift
- 38: Führungsbuchse

- 40: Hohlzylinder
- 42: Durchgangsöffnungen

## Patentansprüche

1. Vorrichtung zur Lagekontrolle eines Venen-Katheters mittels EKG-Ableitung über eine in dem Katheter enthaltene elektrisch leitfähige Flüssigkeit, mit einem an das proximale Ende des Venen-Katheters anschließbaren Drei-Wegehahn und mit einem Kontaktteil, das mit der Flüssigkeit in Berührung kommt und einen Anschluß für eine Patientenleitung eines EKG-Gerätes aufweist,
**dadurch gekennzeichnet, daß** das Kontaktteil (26) in dem Durchgang (20, 34) des Hahnkükens (18) des Drei-Wegehahns angeordnet ist und abgedichtet aus dem Hahnküken (18) zu dem Anschluß (36) geführt ist.

2. Vorrichtung nach Anspruch 1,
**dadurch gekennzeichnet, daß** der Anschluß (36) als Steckverbinder ausgebildet ist.

3. Vorrichtung nach Anspruch 2,
**dadurch gekennzeichnet, daß** der Steckverbinder einen an dem Drei-Wegehahn angeordneten Steckerstift (36) aufweist.

4. Vorrichtung nach einem der Ansprüche 1 bis 3,
**dadurch gekennzeichnet, daß** das Hahnküken (18) eine achsparallele Innenbohrung (24) aufweist, die mit den Durchgangsöffnungen (20) des Hahnkükens (18) in Verbindung steht und daß das Kontaktteil (26) in die Innenbohrung (24) eingesetzt ist.

5. Vorrichtung nach Anspruch 4,
**dadurch gekennzeichnet, daß** der Steckerstift (36) einstückig mit dem Kontaktteil (26) ausgebildet ist und daß der Steckerstift (36) gegen den Durchgang (20, 34) des Hahnkükens (18) abgedichtet ist.

6. Vorrichtung nach Anspruch 4 oder 5,
**dadurch gekennzeichnet, daß** das Kontaktteil (26) einen Stift (27) aufweist, der mittels zweier axial beabstandeter Bundscheiben (28) in der Innenbohrung (24) des Hahnkükens (18) gehalten ist, wobei die Bundscheiben (28) an ihrem Außenumfang gegen die Wandung der Innenbohrung (24) abgedichtet sind, daß zwischen den Bundscheiben (28) eine dem Durchgang des Hahnkükens (18) bildende Ringkammer (34) abgegrenzt ist und daß das Kontaktteil (26) einen einstückig über die eine Bundscheibe (28) hinausragenden Steckerstift (36) aufweist.

7. Vorrichtung nach Anspruch 4 oder 5,
**dadurch gekennzeichnet, daß** das Kontaktteil (26) einen koaxial in die Innenbohrung (24) eingesetzten Hohlzylinder (40) aufweist, der in seinem Mantel mit Durchgangsöffnungen (42) versehen ist, welche mit den Durchgangsöffnungen (20) des Hahnkükens (18) zur Deckung kommen, und daß an dem Hohlzylinder (40) der Steckerstift (36) einstückig axial angeformt ist.

8. Vorrichtung nach einem der Ansprüche 4 bis 7,
**dadurch gekennzeichnet, daß** das Kontaktteil (26) durch eine Führungsbuchse (38) in der Innenbohrung (24) gehalten wird.

## Claims

1. A device for controlling the position of a vein catheter by means of an ECG lead via an electrically conductive liquid contained in the catheter, having a three-way cock that can be connected to the proximal end of the vein catheter and having a contact part which comes into contact with the liquid and a connection for a patient lead of an ECG device,
**characterised in that** the contact part (26) is disposed in the passage (20, 34) of the cock plug (18) of the three-way cock and is passed in sealed manner out of the cock plug (18) to the connection (36).

2. A device according to Claim 1,
**characterised in that** the connection (36) is constructed as a plug connector.

3. A device according to Claim 2,
**characterised in that** the plug connector comprises a connector pin (36) disposed on the three-way cock.

4. A device according to one of Claims 1 to 3,
**characterised in that** the cock plug (18) comprises an axially parallel inner bore (24), which communicates with the through-apertures (20) of the cock plug (18) and **in that** the contact part (26) is inserted into the inner bore (24).

5. A device according to Claim 4,
**characterised in that** the connector pin (36) is constructed in one piece with the contact part (26) and **in that** the connector pin (36) is sealed from the passage (20, 34) of the cock plug (18).

6. A device according to Claim 4 or 5,
**characterised in that** the contact part (26) comprises a pin (27), which is retained by means of two axially spaced flanged disks (28) in the inner bore (25) of the cock plug (18), the flanged disks (28) being sealed on their outer periphery from the wall of the inner bore (24),
**in that** an annular chamber (34) forming the passage of the cock plug (18) is delimited between the flanged disks (28) and **in that** the contact part (26) comprises a connector pin (36) which projects in one piece beyond the one flanged disk (28).

7. A device according to Claim 4 or 5,
**characterised in that** the contact part (26) comprises a hollow cylinder (40) which is inserted coaxially into the inner bore (24) and is provided in its shell with through-apertures (42), which come to be congruent with the through-apertures (20) of the cock plug (18),
and **in that** the connector pin (36) is axially moulded in one piece on the hollow cylinder (40).

8. A device according to one of Claims 4 to 7,
**characterised in that** the contact part (26) is retained by a guide bush (38) in the inner bore (24).

## Revendications

1. Dispositif de contrôle de position d'un cathéter de veine à l'aide d'une ligne de sortie (ECG) par un liquide conducteur électrique placé dans le cathéter, ayant un robinet à trois voies relié à l'extrémité proximale du cathéter de veine et une pièce de contact venant en contact avec le liquide et un branchement pour la ligne de patient de l'appareil (ECG),
**caractérisé en ce que**
la pièce de contact (26) est installée dans le passage (20, 34) du boisseau (18) du robinet à trois voies et cette ligne sort de manière étanche du boisseau (18) pour être reliée au branchement (36).

2. Dispositif selon la revendication 1,
**caractérisé en ce que**
le branchement (36) est un connecteur à fiches.

3. Dispositif selon la revendication 2,
**caractérisé en ce que**
le connecteur comporte une broche de connexion (36) installée sur le robinet à trois voies.

4. Dispositif selon l'une des revendications 1 à 3,
**caractérisé en ce que**
le boisseau (18) comporte un perçage intérieur (24) d'axe parallèle, en liaison avec les orifices traversant (20) du boisseau (18) et la pièce de contact (26) est placée dans le perçage intérieur (24).

5. Dispositif selon la revendication 4,
**caractérisé en ce que**
la broche de connecteur (36) fait corps avec la pièce de contact (26) ; et la broche de connecteur (36) est étanche par rapport au passage (20, 34) du boisseau (18).

6. Dispositif selon la revendication 4 ou 5,
**caractérisé en ce que**
la pièce de contact (26) comporte une broche (27) maintenue à l'aide de deux rondelles à collerette (28) écartées axialement, dans le perçage intérieur (24) du boisseau (18) ;
les rondelles (28) étant rendues étanches par leur périphérie extérieure vis à vis de la paroi du perçage intérieur (24) ; et
entre les rondelles (28) on forme une chambre annulaire (34) constituant le passage du boisseau (18) et la pièce de contact (26) comporte une broche de connecteur (36) en une seule pièce, venant en saillie par rapport à une rondelle (28).

7. Dispositif selon la revendication 4 ou 5,
**caractérisé en ce que**
la pièce de contact (26) comporte un cylindre creux (40) installé coaxialement au perçage intérieur (24), ce cylindre ayant dans son enveloppe des orifices traversant (42) venant coïncider avec les orifices traversant (20) du boisseau (18) et le cylindre creux (40) comporte une broche de connecteur (36) formée axialement en une seule pièce.

8. Dispositif selon l'une des revendications 4 à 7,
**caractérisé en ce que**
la pièce de contact (26) est tenue dans le perçage intérieur (24) par un manchon de guidage (38).
